# EUROPEAN PATENT APPLICATION

(11) **EP 4 462 358 A1**
(43) Date of publication of application: **13.11.2024**
(21) Application number: 24170287.7
(22) Date of filing: 15.04.2024
(51) Int. Cl.: G06T 7/00, G06T 7/11

(54) **METHOD AND APPARATUS FOR IMPROVING PERFORMANCE OF DEEP LEARNING MODEL**

(30) Priority: 09.05.2023 KR 20230059987
(71) Applicant: Medicalip Co., Ltd., Gangwon-do 24341 (KR)
(72) Inventor: PARK, Sang Joon, Seoul (KR); YOON, Soon Ho, Seoul (KR); LEE, Seo Woo, Chuncheon-si, Gangwon-do (KR)
(74) Representative: Patentanwälte Ruff, Wilhelm, Beier, Dauster & Partner mbB

(57) **Abstract**

Provided are a method and apparatus (500) for improving the performance of a deep learning mode (530). The apparatus for improving the performance of a deep learning model prepares a medical image and at least one segmented image obtained by segmenting at least one human tissue from the medical image and trains the deep learning model by respectively inputting the medical image and the at least one segmented image to a plurality of channels.

## Description

The invention relates to a deep learning model used in the medical field to diagnose a lesion, and more particularly, to a method and apparatus for improving the performance of a deep learning model in the medical field. This application is based on and claims priority to Korean Patent Application No. 10-2023-0059987, filed on May 9, 2023, in the Korean Intellectual Property Office, the disclosure of which is incorporated by reference herein in its entirety.

There are various image-based deep learning models. For example, deep learning models for various purposes may be developed based on various images from ImageNet. In the medical field, deep learning models are also used to diagnose lesions from medical images. Most image-based deep learning models are trained by inputting different color scale images (e.g., red, green, and blue (RGB) images) to multiple channels. However, because X-ray images, computed tomography (CT) images, and magnetic resonance imaging (MRI) images are all gray scale images, performance may be degraded when existing deep learning models for image processing are applied to the medical field. For example, because an existing deep learning model that processes color images receives and processes color images through RGB channels including three channels, when a deep learning model in the medical field is generated based on the color images, the same gray scale medical image should be input to the RGB channels. That is, because a deep learning model trained based on different color scales of multiple channels is trained by inputting a single gray scale medical image to the multiple channels, the performance of the deep learning model is degraded.

It is the technical problem underlying the invention to provide a method and apparatus for improving the performance of a deep learning model which method and apparatus show advantages over the prior art.

The invention solves this problem by providing a method having the features of claim 1 and an apparatus having the features of claim 9. Additional aspects will be set forth in part in the description which follows and, in part, will be apparent from the description, or may be learned by practice of the presented embodiments. Advantageous embodiments of the invention are mentioned in the dependent claims the wording of which is herewith incorporated into the description to avoid unnecessary text repetition.

Provided are thus a method and apparatus for improving the performance of a medical deep learning model when the medical deep learning model is generated based on a deep learning model including a plurality of channels.

Additional aspects will be set forth in part in the description which follows and, in part, will be apparent from the description, or may be learned by practice of the presented embodiments.

According to an aspect of the invention, a method of improving performance of a deep learning model including a plurality of channels includes preparing a medical image and at least one segmented image obtained by segmenting at least one human tissue from the medical image, and training the deep learning model by respectively inputting the medical image and the at least one segmented image to the plurality of channels.

According to another aspect of the invention, an apparatus for improving performance of a deep learning model includes an image preparation unit configured to prepare a medical image and at least one segmented image obtained by segmenting at least one human tissue from the medical image, and a training unit configured to train the deep learning model by respectively inputting the medical image and the at least one segmented image to a plurality of channels.

The above and other aspects, features, and advantages of the invention will be more apparent from the following description of exemplary embodiments explained in conjunction with the accompanying drawings, in which:
FIG. 1 is a diagram illustrating an example of a deep learning model, according to an embodiment;
FIG. 2 is a flowchart illustrating an example of a method of improving the performance of a deep learning model, according to an embodiment;
FIG. 3 is a diagram illustrating an example of a method of generating a segmented image, according to an embodiment;
FIG. 4 is a diagram illustrating an example of a method of training a deep learning model, according to an embodiment; and
FIG. 5 is a diagram illustrating a configuration of an example of a performance improvement apparatus, according to an embodiment.

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

A method and apparatus for improving the performance of a deep learning model according to an embodiment will now be described in detail with reference to the accompanying drawings.

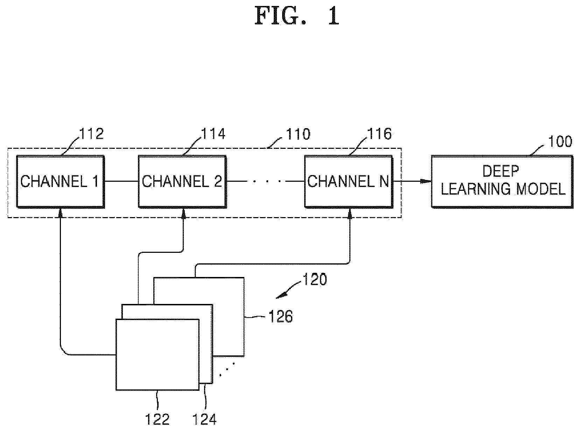

FIG. 1 is a diagram illustrating an example of a deep learning model, according to an embodiment.

Referring to FIG. 1, a deep learning model 100 includes a plurality of channels 116. For example, the deep learning model 100 for processing a color image may include three channels 110 including an R channel, a G channel, and a B channel. Channel images 122, 124, and 126 obtained by dividing values of pixels of a color image 120 into three RGB values are respectively input to channels 112, 114, and 116. According to an embodiment, the number of channels of input data used as an input to the deep learning model 100 may be changed in various ways. However, for convenience of explanation, the following will be described assuming that there are three channels such as RGB channels.

The deep learning model 100 may be implemented with any of various artificial neural networks such as a convolutional neural network (CNN) or U-Net, and is not limited to a specific example. The deep learning model 100 may be a model created for various purposes by using a medical image or a non-medical image (e.g., an image of an image database of ImageNet). For example, the deep learning model 100 may be a model created to identify or classify a specific object (e.g., a dog or a cat) in a color image. Alternatively, the deep learning model 100 may be a model created to segment a specific human tissue (e.g., lung or lesion) or diagnose a lesion (e.g., emphysema, pulmonary edema, pneumonia, or various cancers) in the medical field. In another example, the deep learning model 100 may be a newly created model for the present embodiment. In addition, the deep learning model 100 used in the present embodiment may be any of various types of models that receive and process an image, and is not limited to a specific example.

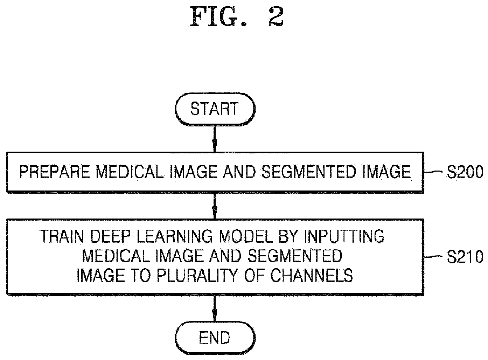

The present embodiment proposes a method of training and generating the deep learning model 100, which is an existing model or a new model, so that the deep learning model 100 is optimized for processing a medical image, which will be described in detail with reference to FIG. 2.

FIG. 2 is a flowchart illustrating an example of a method of improving the performance of a deep learning model, according to an embodiment.

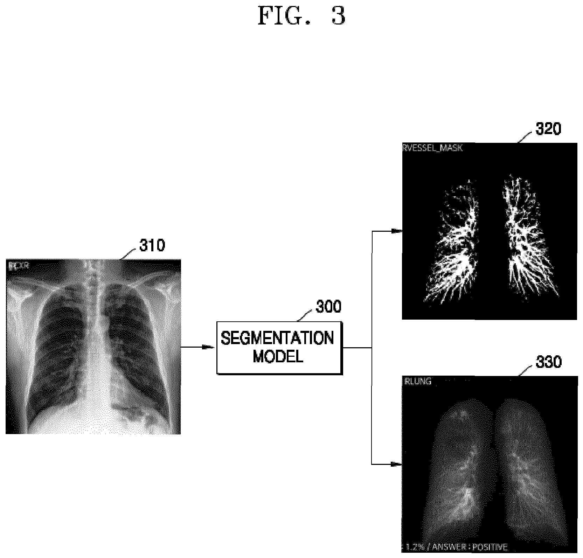

Referring to FIG. 2, a performance improvement apparatus prepares a medical image and at least one segmented image obtained by segmenting at least one human tissue (e.g., lung, heart, blood vessel, muscle, fat, or lesion) from the medical image (S200). In an embodiment, the performance improvement apparatus may receive the medical image and the segmented image from an external device. In another embodiment, when the performance improvement apparatus receives the medical image from the external device, the performance improvement apparatus may generate the segmented image from the medical image. An example of a method of generating an image obtained by segmenting a human tissue from a medical image will be described below with reference to FIG. 3.

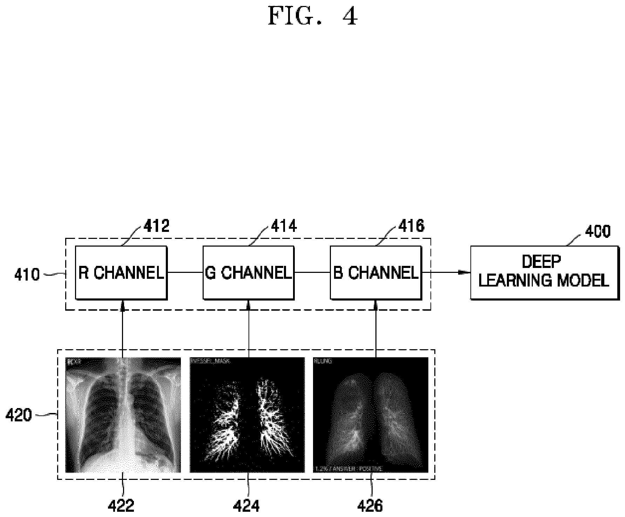

The performance improvement apparatus trains a deep learning model by respectively inputting the medical image and the at least one segmented image to a plurality of channels of the deep learning model. For example, when the deep learning model includes RGB channels, the performance improvement apparatus inputs the medical image and two segmented images generated by segmenting a human tissue from the medical image to the RGB channels. An example of a method of inputting and training a medical image and a segmented image to a deep learning model including RGB channels is illustrated in FIG. 4.

FIG. 3 is a diagram illustrating an example of a method of generating a segmented image, according to an embodiment.

Referring to FIG. 3, a segmentation model 300 is a model for segmenting at least one human tissue from a medical image 310. The medical image 310 may be a two-dimensional (2D) medical image such as an X-ray image.

The segmentation model 300 may be created with any of various conventional architectures such as a CNN. In an example, in order to improve the performance of the segmentation model 300, the segmentation model 300 may be trained by using training data made based on a three-dimensional (3D) medical image. The 3D medical image may be a CT image or an MRI image.

In more detail, a performance improvement apparatus segments a specific human tissue (e.g., lung, heart, blood vessel, or lesion) by applying any of various conventional segmentation algorithms (e.g., deep learning models for 3D segmentation) to a 3D medical image. In a 2D medical image such as an X-ray image, because various tissues are overlapped and displayed on a 2D plane, it may be difficult to accurately segment a specific human tissue from the 2D medical image and segmentation accuracy may be low. Accordingly, in the present embodiment, a method of segmenting a specific human tissue from a 3D medical image is used. The specific human tissue segmented from the 3D medical image is 3D data (i.e., voxels). The performance improvement apparatus generates a 2D medical image by two-dimensionally projecting the human tissue (e.g., voxel data) segmented from the 3D medical image. Also, the performance improvement apparatus generates a 2D medical image by two-dimensionally projecting the 3D medical image itself. When the 3D medical image itself is two-dimensionally projected, because brightness values of a plurality of tissues are all overlapped and reflected on a plane, an image in which the plurality of tissues are overlapped, like an X-ray image, may be made.

The performance improvement apparatus generates training data including an image (hereinafter, referred to as a basic image) made by two-dimensionally projecting a 3D medical image and an image (hereinafter, referred to as a tissue image) made by two-dimensionally projecting a 3D image of a specific human tissue. The performance improvement apparatus may train the segmentation model 300 through a supervised learning method by using the training data in which the basic image is labeled with the tissue image (i.e., ground truth). When a 2D medical image captured by an X-ray device is input to the segmentation model 300 that has been trained, the segmentation model 300 outputs first and second segmented images 320 and 330 obtained by segmenting a specific region from the 2D medical image.

The segmentation model 300 may be trained to segment a plurality of human tissues from the medical image 310. For example, the segmentation model 300 may be a model that, when receiving a chest X-ray image, outputs the first segmented image 320 of a blood vessel region and the second segmented image 330 of a lung region. In another example, the segmentation model 300 may be a model that, when receiving a chest X-ray image, outputs a first segmented image of a lung region and a second segmented image of a heart region. In addition, the type and number of human tissues segmented by the segmentation model 300 may be changed in various ways according to which anatomical region is mainly affected by a disease to be diagnosed or classified. For example, emphysema darkens the lungs and reduces blood vessels on an X-ray image of an emphysema region. In another example, there may be a plurality of segmentation models that segment different human tissues from the medical image 310.

FIG. 4 is a diagram illustrating an example of a method of training a deep learning model, according to an embodiment.

Referring to FIG. 4, a deep learning model 400 includes RGB channels 410. A performance improvement apparatus may train the deep learning model 400 to diagnose a lesion (e.g., emphysema or pulmonary edema) from a medical image. The deep learning model 400 may be an existing model or a newly created model for the present embodiment.

In an embodiment, a process of training the deep learning model 400 to diagnose emphysema will be described. The performance improvement apparatus prepares two segmented images 424 and 426 obtained by segmenting a blood vessel region and a lung region from a medical image 422. The segmented images 424 and 426 may be provided from the an external device or may be directly generated by the performance improvement apparatus by using the segmentation model 300 of FIG. 3. The performance improvement apparatus trains the deep learning model 400 by using training data in which a training image 420 including the medical image 422 and the two segmented images 424 and 426 is labeled with whether there is emphysema. The medical image 422 and the two segmented images 424 and 426 are respectively input to channels 412, 414, and 416 of the deep learning model 400.

In another embodiment, a process of training a deep learning model to diagnose pulmonary edema will be described. The performance improvement apparatus prepares two segmented images obtained by segmenting a lung region and a heart region from a medical image. The segmented images may be provided from an external device or may be directly generated by the performance improvement apparatus by using the segmentation model 300 of FIG. 3. The performance improvement apparatus trains the deep learning model by using training data in which a training image including the medical image and the two segmented images is labeled with whether there is pulmonary edema. The medical image and the two segmented images are respectively input to the RGB channels of the deep learning model 400.

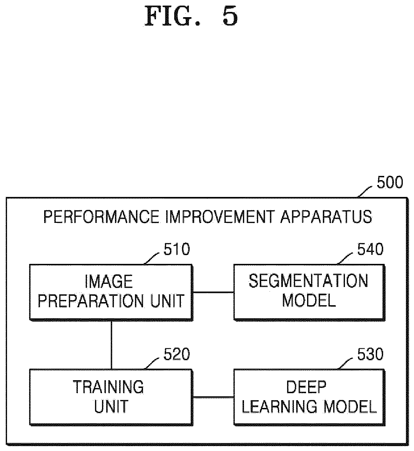

FIG. 5 is a diagram illustrating a configuration of an example of a performance improvement apparatus, according to an embodiment.

Referring to FIG. 5, a performance improvement apparatus 500 includes an image preparation unit 510, a training unit 520, a deep learning model 530, and a segmentation model 540. According to an embodiment, the segmentation model 540 may be omitted. However, the following will be described assuming that the segmentation 540 is included. In an embodiment, the performance improvement apparatus 500 may be implemented as a computing device including a memory, a processor, and an input/output device. In this case, each element may be implemented as software, may be loaded into the memory, and then may be executed by the processor.

The image preparation unit 510 prepares a medical image and at least one segmented image obtained by segmenting at least one human tissue from the medical image. The image preparation unit 510 may receive the medical image and the segmented image from an external device. In another embodiment, when the image preparation unit 510 receives the medical image, the image preparation unit 510 may generate the at least one segmented image from the medical image through the segmentation model 540. An example of a method of generating a segmented image by using the segmentation model 540 is illustrated in FIG. 3.

The training unit 520 trains the deep learning model 530 by respectively inputting the medical image and the at least one segmented image to a plurality of channels of the deep learning model 530. For example, when the deep learning model 530 for diagnosing emphysema is to be created, the image preparation unit 510 prepares segmented images of a lung region and a blood vessel region. The training unit 520 trains the deep learning model 530 to determine whether there is emphysema by using the medical image and the segmented image. An example of a method by which the training unit 520 trains the deep learning model 530 is illustrated in FIG. 4.

The invention may also be implemented as computer-readable code on a computer-readable recording medium. The computer-readable recording medium includes any storage device that may store data which may be thereafter read by a computer system. Examples of the computer-readable recording medium include a read-only memory (ROM), a random-access memory (RAM), a compact disk (CD)-ROM, a magnetic tape, a floppy disk, and an optical data storage device. The computer-readable recording medium may also be distributed over network-coupled computer systems so that the computer-readable code is stored and executed in a distributive manner.

The invention has been described with reference to the embodiments thereof. It will be understood by one of ordinary skill in the art that various changes in form and details may be made therein without departing from the spirit and scope of the invention as defined by the appended claims. Hence, the disclosed embodiments should be considered in descriptive sense only and not for purposes of limitation. The scope of the invention is defined only by the following claims, and all the equivalents of the embodiments may also be construed to be in the scope of the invention.

According to an embodiment, when a deep learning model including a plurality of channels is applied to the medical field, the performance of the deep learning model may be improved. In an embodiment, an existing deep learning model trained based on a non-medical color image may be retrained with a medical image to generate a medical deep learning model. In another example, the performance of an existing deep learning model including a plurality of channels used in the medical field may be improved. In another example, the invention may also be applied to a multimodal large language model (LLM).

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by one of ordinary skill in the art that various changes in form and details may be made therein without departing from the spirit and scope of the invention as defined by the following claims.

## Claims

1. Method of improving performance of a deep learning model comprising a plurality of channels, the method comprising:
preparing a medical image and at least one segmented image obtained by segmenting at least one human tissue from the medical image; and
training the deep learning model by respectively inputting the medical image and the at least one segmented image to the plurality of channels.

2. Method of claim 1, wherein the deep learning model is an image processing model comprising RGB channels.

3. Method of claim 1 or 2, wherein the deep learning model is a model trained based on a non-medical image.

4. Method of any of claims 1 to 3, wherein the medical image is a gray scale X-ray image.

5. Method of any of claims 1 to 4, wherein the deep learning model is a model for diagnosing a lesion based on a medical image.

6. Method of any of claims 1 to 5, wherein
the medical image is a chest X-ray image, and
the at least one segmented image comprises a first segmented image of a lung region and a second segmented image of a blood vessel region segmented from the medical image.

7. Method of any of claims 1 to 5, wherein
the medical image is a chest X-ray image, and
the at least one segmented image comprises a first segmented image of a lung region and a second segmented image of a heart region segmented from the medical image.

8. Method of any of claims 1 to 7, wherein the preparing of the at least one segmented image comprises segmenting the at least one human tissue from the medical image by using a segmentation model trained based on a projection image generated by two-dimensionally projecting a three-dimensional (3D) medical image.

9. Apparatus for improving performance of a deep learning model (530), the apparatus comprising:
an image preparation unit (510) configured to prepare a medical image and at least one segmented image obtained by segmenting at least one human tissue from the medical image; and
a training unit (520) configured to train the deep learning model by respectively inputting the medical image and the at least one segmented image to a plurality of channels.

10. Computer-readable recording medium having recorded thereon a computer program for performing the method of any of claims 1 to 8.
